(19) Europäisches Patentamt
European Patent Office
Office européen des brevets

(11) **EP 3 322 524 B1**

(12) **FASCICULE DE BREVET EUROPEEN**

(45) Date de publication et mention de la délivrance du brevet:
**14.04.2021 Bulletin 2021/15**

(21) Numéro de dépôt: **16739054.1**

(22) Date de dépôt: **05.07.2016**

(51) Int Cl.:
*B01J 23/20* (2006.01)    *B01J 21/08* (2006.01)
*B01J 35/10* (2006.01)    *B01J 37/02* (2006.01)
*B01J 37/06* (2006.01)    *C07C 11/167* (2006.01)
*C07C 1/20* (2006.01)

(86) Numéro de dépôt international:
**PCT/EP2016/065823**

(87) Numéro de publication internationale:
**WO 2017/009107 (19.01.2017 Gazette 2017/03)**

(54) **PROCEDE DE PREPARATION D'UN CATALYSEUR A BASE DE TANTALE DEPOSE SUR SILICE POUR LA TRANSFORMATION DE L'ETHANOL EN BUTADIENE**

HERSTELLUNGSVERFAHREN EINES KATALYSATORS BESTEHEND AUS TANTAL AUF EINEM SILIKATRÄGER ZUR HERSTELLUNG VON BUTADIEN AUS ETHANOL

PREPARATION PROCESS OF A SILICA SUPPORTED CATALYST COMPRISING TANTALUM FOR THE TRANSFORMATION OF ETHANOL INTO BUTADIENE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2015 FR 1556662**

(43) Date de publication de la demande:
**23.05.2018 Bulletin 2018/21**

(73) Titulaires:
• **IFP Energies nouvelles**
**92500 Rueil-Malmaison (FR)**
• **Compagnie Générale des Etablissements Michelin**
**63000 Clermont-Ferrand (FR)**

(72) Inventeurs:
• **CADRAN, Nicolas**
**69600 Oullins (FR)**
• **CHAUMONNOT, Alexandra**
**69008 Lyon (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
WO-A1-2014/209682    WO-A1-2017/009108
KR-A- 20140 047 329    KR-A- 20140 050 531
US-A1- 2013 289 133

• V.M. Gun'ko ET AL: "Structural and Energetic Characteristics of Silicas Modified by Organosilicon Compounds", Journal of Colloid and Interface Science, vol. 249, no. 1, 1 May 2002 (2002-05-01), pages 123-133, XP055473381, US ISSN: 0021-9797, DOI: 10.1006/jcis.2002.8259
• Tae-Wan Kim ET AL: "Butadiene production from bioethanol and acetaldehyde over tantalum oxide-supported spherical silica catalysts for circulating fluidized bed", Chemical Engeneering Journal, vol. 278, 1 October 2015 (2015-10-01), pages 217-223, XP055276656, AMSTERDAM, NL ISSN: 1385-8947, DOI: 10.1016/j.cej.2014.09.110
• Ho-Jeong Chae ET AL: "Butadiene production from bioethanol and acetaldehyde over tantalum oxide-supported ordered mesoporous silica catalysts", Applied Catalysis B: Environmental, vol. 150-151, 1 May 2014 (2014-05-01), pages 596-604, XP055276659, AMSTERDAM, NL ISSN: 0926-3373, DOI: 10.1016/j.apcatb.2013.12.023

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**État de la technique antérieure**

Le butadiène est largement utilisé dans l'industrie chimique notamment en tant que réactif pour la production de polymères. Actuellement, le butadiène est presque entièrement produit à partir d'unités de vapocraquage dont il constitue un sous-produit valorisable. La fluctuation du prix du pétrole et la demande toujours plus importante de cet intermédiaire chimique ont rendu son prix très volatil, ce qui incite à une diversification des moyens d'approvisionnement. Il est ainsi bien connu de l'Homme du métier que le 1,3-butadiène peut être produit à partir d'éthanol. Deux procédés ont été industrialisés à grande échelle : le "S. K. Process" et le "Carbide Process". Dans le "S. K. Process", le 1,3-butadiène est produit à partir d'éthanol en une étape, alors que dans le "Carbide Process", le 1,3-butadiène est produit en deux étapes : de l'éthanol est tout d'abord converti en acétaldéhyde, puis un mélange éthanol-acétaldéhyde est converti en 1,3-butadiène. La principale distinction entre les catalyseurs mis en jeu dans ces procédés est que l'un (SK Process) est capable de déshydrogéner l'éthanol en acétaldéhyde tout en produisant du butadiène à partir du mélange ainsi formé alors que l'autre ne l'est pas d'où la nécessité d'une première étape de déshydrogénation sur un catalyseur spécifique. Les éléments chimiques constitutifs du catalyseur les plus efficaces pour cette méthode de production du butadiène sont le magnésium, le tantale, le zirconium, le hafnium, avec des sélectivités en butadiène situées entre 50 et 69%, le niobium (ou colombium) étant considéré comme un élément peu attractif avec des sélectivités inférieures à 40% (B. B. Corson, H. E. Jones, C. E. Welling, J. A. Hinckley, E. E. Stahly, Ind. Eng. Chem., 1950, 42 (2), p 359-373).

Quel que soit le procédé (une ou deux étapes), le bilan global de la réaction principale s'écrit comme suit :

$$2\ CH_3CH_2OH \rightleftarrows CH_2CHCHCH_2 + H_2 + 2H_2O$$

Derrière ce bilan global se cachent de nombreuses réactions chimiques comprenant une réaction de déshydrogénation permettant de générer de l'acétaldéhyde (I), une réaction d'aldolisation/crotonisation de l'acétaldéhyde en crotonaldéhyde (II), une réaction de Merwein-Pondorff-Verley (MPV) entre l'éthanol et le crotonaldéhyde (III) et finalement une étape de déshydratation de l'alcool crotylique en butadiène (IV).

$$I : \quad CH_3CH_2OH \rightleftarrows CH_3CHO + H_2$$

$$II : \quad 2\ CH_3CHO \rightleftarrows CH_3CHCH\text{-}CHO + H_2O$$

$$III : \quad CH_3CHCH\text{-}CHO + CH_3CH_2OH \rightleftarrows CH_3CHCH\text{-}CH_2OH + CH_3CHO$$

$$IV : \quad CH_3CHCH\text{-}CH_2OH \rightleftarrows CH_2CHCHCH_2 + H_2O$$

Cette multiplicité de réactions chimiques est à l'origine de nombreux sous-produits si l'enchainement des étapes ne se fait pas dans l'ordre précisé ci-dessus, avec notamment la présence de réactions de déshydratation et de condensation secondaires. De plus, d'autres réactions peuvent se produire (comme l'isomérisation, la cyclisation, la réaction de Diels et Alder, etc.) augmentant encore le nombre de sous-produits. A ce stade, notons que, suivant la nature du catalyseur mis en œuvre pour la transformation de l'éthanol (ou du mélange éthanol-acétaldéhyde) en 1,3-butadiène, la répartition desdits sous-produits peut fortement évoluer. Ainsi, l'ajout d'un élément acide fera augmenter la production de produits de déshydratation (par exemple l'éthylène ou le diéthyléther) tandis que l'ajout d'un élément basique favorisera la formation de produits de condensation multiples (par exemple les hexènes ou les hexadiènes).

Conséquemment, quel que soit le procédé (une ou deux étapes), la sélectivité de la transformation de l'éthanol (ou du mélange éthanol-acétaldéhyde) en 1,3-butadiène est modérée. Or, en raison du prix relativement élevé de la matière première, l'étude économique du procédé montre que l'efficacité de la transformation de la charge constitue un levier important pour assurer sa viabilité. De nombreux efforts ont donc été déployés pour maximiser cette sélectivité.

En particulier, lors du développement du procédé de production de butadiène à partir d'un mélange éthanol/acétaldéhyde (procédé en deux étapes), le meilleur catalyseur trouvé a été un oxyde de tantale déposé sur une silice amorphe (Ind. Eng. Chem., 1949, 41, p 1012-1017). La sélectivité en butadiène était de 69% pour une conversion initiale de la charge de 34%. Il a également été montré que l'emploi de ce même catalyseur dans une unité industrielle "Carbide" conduisait à la formation des impuretés (sous-produits) majoritaires suivantes : diéthyléther (23% poids des impuretés), éthylène (11% poids des impuretés), hexènes, hexadiènes (11% poids des impuretés), etc. (W. J. Toussaint, J. T. Dunn, D. R. Jackson, Industrial and Engineering Chemistry, 1947, 39 (2), p 120-125). Malgré la présence de sous-produits, leur formation est limitée par les propriétés d'acidobasicité relativement faibles de l'élément tantale. Ce dernier permet également de catalyser très efficacement les réactions II, III et IV. Un de ses uniques inconvénients réside dans son prix.

En effet, d'après le rapport écrit en 2012 par Jonathan Burla, Ross Fehnel, Philip Louie et Peter Terpeluk de l'université de Pennsylvanie et intitulé «TWO-STEP PRODUCTION OF 1,3-BUTADIENE FROM ETHANOL », le prix de la silice se situe autour de $0,96/lb et celui du tantale autour de $162/lb. A titre indicatif, les cours actuels du niobium et du zirconium se situent quant à eux autour de $20/lb et $1/lb, soit environ un rapport de prix d'un ordre de grandeur entre le niobium et le tantale et de deux ordres de grandeur entre le zirconium et le tantale.

Diverses études ont alors été réalisées pour optimiser l'efficacité du tantale et/ou substituer cet élément. Le brevet US 2421361 (W. J. Toussaint, J. T. Dunn, Carbide and Carbon Chemical Corporation, 1947) décrit, quant à lui, un procédé pour la préparation de butadiène qui comprend la transformation d'un aldéhyde monooléfinique acyclique (crotonaldéhyde ou acétaldéhyde) et d'un alcool monohydroxylé (éthanol) sur un catalyseur du groupe de l'oxyde de zirconium, l'oxyde de tantale, l'oxyde de niobium et l'une des combinaisons de ces oxydes avec de la silice. Cependant, d'après les exemples fournis, l'oxyde de tantale utilisé seul reste le meilleur catalyseur pour convertir le mélange spécifique éthanol/acétaldéhyde. D'après Ind. Eng. Chem., 1950, 42 (2), p 359-373, les meilleures associations pour la transformation du mélange éthanol/acétaldéhyde sont : Ta-Cu, Ta-Zr, Zr-Nb, Zr-Ti et Zr-Th déposés sur un support silicique (brevets US2374433, US2436125, US2438464, US2357855, US2447181). Plus récemment, la plupart des études récentes ont cherché à éliminer complétement le tantale de la formulation catalytique, notamment grâce à l'utilisation de l'élément zirconium ou magnésium :

- la demande WO 2014/199349 (BASF) utilise une association Zr, Zn, Cu,
- la demande WO 2014/180778 (Synthos) revendique une association Zr, Zn, La,
- la demande WO 2014/049158 (Lanxess) utilise un oxyde mixte Mg-Si dopé par des éléments comme Ti, V, Mo, Mn, Cu, Ni, Zn ou Cr,
- la demande WO 2013/125389 (Daicel) revendique l'utilisation d'un oxyde mixte Mg-Si dopé par un métal appartenant aux colonnes 4 à 13,
- la demande WO 2012/015340 (Unisit) utilise l'association d'un élément à l'état métallique de la colonne 11 et d'un oxyde métallique choisi parmi le magnésium, le titane, le zirconium, le tantale et le niobium.

Notons que, quel que soit l'axe de recherche choisi par l'Homme du métier pour améliorer les performances du catalyseur considéré, le support de choix pour l'ensemble des matériaux étudiés est un support silicique mésoporeux. Quelques études se sont donc concentrées sur l'amélioration du support. Ainsi, la demande WO 2014/061917 cherche à améliorer le catalyseur à base de tantale via l'utilisation d'un support silicique caractérisé par des mésopores de taille et de morphologie uniformes et répartis de façon périodique au sein du matériau (silice dite mésostructurée).

Le lavage acide de silices est une méthode connue de l'Homme de l'art pour modifier les performances de colonnes chromatographiques en éliminant certaines impuretés indésirables (J. Nawrocki, Chromatographia, 1991, 31 (3/4), « Silica Surface Controversies, Strong Adsorption Sites, their Blockage and Removal. Part I »). En catalyse, cette technique été utilisée dans le cas de la métathèse des oléfines (US2011196185A (UOP LLC)) en présence d'un catalyseur comprenant du tungstène dispersé sur un support comprenant de la silice, permettant d'améliorer l'activité du catalyseur sans dégrader leur sélectivité. Toutefois, cette mise en œuvre particulière n'est pas aisément transposable. Ainsi, par exemple, si la productivité d'un catalyseur Nb-Silice est améliorée lorsque le support est lavé à l'acide, sa sélectivité dans la réaction de production de 1,3-butadiène est fortement dégradée.

## Résumé de l'invention

L'invention concerne un procédé de préparation d'un catalyseur comprenant au moins l'élément tantale, et au moins une matrice oxyde mésoporeuse à base de silice, la masse de tantale représentant entre 0,1 et 30% de la masse de ladite matrice oxyde mésoporeuse, comprenant au moins les étapes suivantes :

a) au moins une étape de lavage acide d'une matrice oxyde mésoporeuse comprenant avant lavage au moins 90% poids de silice, ladite matrice oxyde mésoporeuse à base de silice avant lavage étant une silice amorphe mésoporeuse à porosité non organisée sans micropores ayant une surface spécifique BET d'au moins 250 m$^2$/g, les pourcentages poids étant exprimés par rapport à la masse totale de la matrice oxyde mésoporeuse, ledit lavage étant réalisé via la mise en contact avec au moins un acide inorganique, choisi parmi l'acide nitrique, l'acide sulfurique et l'acide chlorhydrique, dilué dans une solution aqueuse à une concentration comprise dans la gamme de 0,05 M à 3 M, à une température comprise entre 0°C et 120°C et un temps de contact de ladite solution acide avec ladite matrice oxyde mésoporeuse compris entre 10 min et 10 h,

b) au moins une étape de traitement thermique de ladite matrice lavée issue de l'étape a), ledit traitement thermique étant un séchage réalisé à une température de 80 à 300°C durant une période inférieure à 24 h, de façon à obtenir un support de catalyseur,

c) au moins une étape de dépôt d'au moins un précurseur métallique d'au moins l'élément tantale sur la surface dudit support obtenu à l'issue de l'étape b) et

d) au moins une étape de traitement thermique du solide issu de l'étape c), ledit traitement thermique étant un séchage suivi d'une calcination, le séchage étant réalisé sous circulation d'air à une température comprise entre 80 et 150°C pendant une durée comprise entre 1 et 24 heures, et la calcination étant réalisée sous circulation d'un flux gazeux comprenant de l'oxygène, à une température comprise entre 450 et 600°C, pendant une durée comprise entre 1 et 6 heures.

L'invention concerne également un procédé de préparation d'un catalyseur comprenant au moins l'élément tantale, et au moins une matrice oxyde mésoporeuse à base de silice, la masse de tantale représentant entre 0,1 et 30% de la masse de ladite matrice oxyde mésoporeuse, comprenant au moins les étapes suivantes :

a') au moins une étape de dépôt d'au moins un précurseur métallique d'au moins l'élément tantale sur la surface d'une matrice oxyde mésoporeuse comprenant avant lavage au moins 90% poids de silice, ladite matrice oxyde mésoporeuse à base de silice avant lavage étant une silice amorphe mésoporeuse à porosité non organisée sans micropores ayant une surface spécifique BET d'au moins 250 m$^2$/g, les pourcentages poids étant exprimés par rapport à la masse totale de la matrice oxyde mésoporeuse,

b') au moins une étape de traitement thermique du solide issu de l'étape a'), ledit traitement thermique étant un séchage suivi d'une calcination, le séchage étant réalisé sous circulation d'air à une température comprise entre 80 et 150°C pendant une durée comprise entre 1 et 24 heures, et la calcination étant réalisée sous circulation d'un flux gazeux comprenant de l'oxygène, à une température comprise entre 450 et 600°C, pendant une durée comprise entre 1 et 6 heures

c') au moins une étape de lavage acide du solide issu de l'étape b'), avec au moins un acide inorganique, choisi parmi l'acide nitrique, l'acide sulfurique et l'acide chlorhydrique, dilué dans une solution aqueuse à une concentration comprise dans la gamme de 0,05 M à 3 M, à une température comprise entre 0°C et 120°C et un temps de contact de ladite solution acide avec ledit solide compris entre 10 min et 10 h,

d') au moins une étape de traitement thermique du solide issu de l'étape c'), ledit traitement thermique étant un séchage réalisé à une température de 80 à 300°C durant une période inférieure à 24 h.

## Intérêt de l'invention

Par rapport à l'état de l'art mentionné ci-dessus, la présente invention propose une approche originale pour améliorer le potentiel d'un catalyseur de production de butadiène à partir d'un mélange comprenant au moins de l'éthanol, via un prétraitement spécifique et adapté du support de nature silicique communément employé : un lavage acide. Ce dernier n'implique pas uniquement l'élimination de certaines impuretés potentielles du support mais modifie également sa chimie de surface, ce qui conduit à une meilleure activité du catalyseur à base de tantale ainsi qu'à une amélioration de sa sélectivité. Un autre effet est une amélioration de la productivité et de la sélectivité en butadiène à iso-débit de charge.

## Exposé de l'invention

L'invention concerne la préparation d'un catalyseur, utilisé pour la production de butadiène à partir d'une charge comprenant au moins de l'éthanol, comprenant au moins l'élément tantale, susceptible de réaliser au moins les réactions II à IV susmentionnées, et au moins une matrice oxyde mésoporeuse à base de silice ayant subi un lavage acide.

Le catalyseur comprend une matrice oxyde mésoporeuse à base de silice lavée via la mise en contact de ladite matrice avec au moins un acide inorganique, dilué dans une solution aqueuse, et choisi parmi l'acide nitrique, l'acide sulfurique et l'acide chlorhydrique, l'acide nitrique et l'acide chlorhydrique étant préférés. La concentration en acide inorganique utilisé est comprise dans la gamme de 0,05 molaire (M) à 3 M et de façon préférée comprise dans la gamme de 0,1 M à 1 M. Ledit lavage acide peut être effectué dans des conditions statiques (par exemple en « batch ») ou continues (par exemple circulation dans une colonne de lavage avec ou sans recyclage partiel ou total de l'effluent). Selon l'invention, les conditions représentatives de ce lavage à l'acide sont une température comprise entre 0°C et 120°C, préférentiellement entre 15° et 80°C, et de manière très préférée entre 20°C et 70°C. Le temps de contact de la solution acide avec la matrice oxyde mésoporeuse varie entre 10 minutes et 10 heures et de manière préférée entre 30 minutes et 3 heures. Une opération optionnelle de rinçage à l'eau peut ensuite être mise en place de façon à éliminer l'excédent d'acidité. Celle-ci peut être réalisée dans les mêmes conditions que décrites ci-dessus.

Le catalyseur préparé selon l'invention comprend une masse de Ta comprise entre 0,1 et 30%, de préférence entre 0,3 et 10%, de façon préférée entre 0,5 et 5% et de manière très préférée entre 0,5 et 2% de la masse de ladite matrice oxyde mésoporeuse.

Par catalyseur comprenant un élément A, la masse de l'élément A étant comprise, ou représentant entre, x et y% de la masse de la matrice oxyde mésoporeuse, on entend que ledit catalyseur comprend entre x et y parties en poids dudit élément A pour 100 parties en poids de ladite matrice oxyde mésoporeuse.

Le catalyseur préparé selon l'invention comprend avantageusement également au moins un élément choisi dans le groupe constitué par les éléments des groupes 2, 3, 4 et 5 du tableau périodique et leurs mélanges, de préférence choisi dans le groupe constitué par les éléments des groupes 2 et 5 du tableau périodique et leurs mélanges, et de façon encore plus préférée au moins un élément choisi dans le groupe constitué par les éléments Ca et Ba du groupe 2 et Nb du groupe 5 du tableau périodique et leurs mélanges, la masse dudit élément représentant entre 0,01 et 5%, de préférence entre 0,01 et 1%, de façon préférée entre 0,05 et 0,5% de la masse de la matrice oxyde mésoporeuse à base de silice.

Dans un arrangement particulier, le catalyseur préparé selon l'invention comprend avantageusement également au moins un élément choisi dans le groupe constitué par les groupes 11 et 12 du tableau périodique et leurs mélanges, c'est-à-dire de la classification périodique des éléments, de façon plus préférée au moins un élément choisi dans le groupe 12 du tableau périodique et de façon encore plus préférée, l'élément Zn, la masse dudit élément représentant entre 0,5 et 10%, et de préférence entre 1 et 5% de la masse de ladite matrice oxyde mésoporeuse à base de silice. Cet arrangement est particulièrement avantageux dans le cas où le catalyseur préparé selon l'invention est utilisé dans un procédé une étape, c'est-à-dire dans un procédé traitant une charge comprenant principalement de l'éthanol. Par principalement de l'éthanol, on entend que le ratio massique éthanol sur acétaldéhyde dans ladite charge, quand ladite charge comprend de l'acétaldéhyde, est au moins supérieur à 1, de préférence au moins supérieur à 5, ladite charge pouvant également ne pas comprendre d'acétaldéhyde.

La matrice oxyde mésoporeuse à base de silice ayant subi une étape de lavage acide du catalyseur est dénommée support du catalyseur dans la suite du texte.

Par matrice oxyde mésoporeuse à base de silice, on entend un matrice oxyde mésoporeuse comprenant, avant lavage selon l'invention, au moins 90% poids par rapport à la masse totale de la matrice oxyde mésoporeuse de silice. De préférence, ladite matrice avant lavage comprend de la silice en une quantité d'au moins 95% poids (c'est à dire à partir de 95% jusqu'à 100%) par rapport à la masse totale de la matrice oxyde mésoporeuse, de façon plus préférée d'au moins 98% poids (c'est-à-dire à partir de 98% jusqu'à 100%) et de façon encore plus préférée d'au moins 99,5% poids (c'est-à-dire à partir de 99,5% jusqu'à 100%).

La matrice oxyde mésoporeuse à base de silice avant lavage utilisée selon l'invention contient avantageusement, en plus de la silice, au moins des oxydes faisant partie de la liste non exhaustive suivante : zircone, oxyde de titane, oxyde de bore, oxyde de lanthane, oxyde de cérium. De préférence, la matrice oxyde mésoporeuse avant lavage utilisée selon l'invention contient en plus de la silice de l'oxyde de titane. De façon additionnelle, la matrice oxyde mésoporeuse à base de silice comprend éventuellement au moins un élément faisant partie de la liste non exhaustive suivante : Na, K, Mg, Ca, Al, Cu, Zn, Fe, Ni, Co, S, P. Avantageusement, la matrice oxyde mésoporeuse à base de silice comprend au moins un élément faisant partie du groupe constitué par Na, K, Mg, Ca, Al, Cu, Zn, Fe, Ni, Co, S et P et leurs mélanges. La matrice oxyde mésoporeuse à base de silice avant lavage comprend avantageusement plus de 500 ppm de métaux alcalins.

La matrice oxyde mésoporeuse à base de silice avant lavage est mésoporeuse, c'est-à-dire qu'elle se caractérise par la présence de pores dont la taille varie entre 2 et 50 nm selon la classification de l'IUPAC (K. S. W. Sing, D. H. Everett, R. A. Haul, L. Moscou, J. Pierotti, J. Rouquerol, T. Siemieniewska, Pure Appl. Chem., 1985, 57, 603). En plus d'être mésoporeuse, la matrice oxyde mésoporeuse à base de silice avant lavage est une silice amorphe mésoporeuse à porosité non organisée sans micropores. Pour exemple, on peut utiliser les silices Davisil Grade 636 ou 646, commercialisées par WR Grace k Co., Columbia, Md., dénommées également « gels de silice » car obtenues par précipitation à pH ≤ 7, les silices commerciales dites précipitées, obtenues via un pH ≥ 7, ou encore les silices commerciales dites pyrogénées, obtenues par hydrolyse de $SiCl_4$ à 1000°C. Il est également possible de synthétiser la silice à façon selon les méthodes connues de l'Homme du métier, notamment par utilisation des méthodes de synthèse « traditionnelles » inorganiques (précipitation/gélification à partir de sels dans des conditions douces de température et de pression) ou « modernes » métallo-organiques (précipitation/gélification à partir d'alcoxydes dans des conditions douces de température et de pression). Des résultats particulièrement avantageux sont ainsi obtenus après lavage acide d'une matrice oxyde mésoporeuse à base de silice caractérisée, avant lavage, par une surface spécifique d'au moins 250 $m^2$/g, de façon préférée par une surface spécifique comprise entre 250 $m^2$/g et 700 $m^2$/g et de façon encore plus préférée par une surface spécifique comprise entre 400 $m^2$/g et 600 $m^2$/g. De plus, le diamètre moyen des pores (ou taille de pores) de la matrice oxyde mésoporeuse à base de silice avant lavage utilisée selon l'invention est de préférence d'au moins 4 nm, de façon préférée compris entre 4,5 et 50 nm et de façon encore plus préférée compris entre 4,5 et 20 nm.

De façon globale, le procédé de préparation du catalyseur comprenant au moins l'élément tantale et au moins une matrice oxyde mésoporeuse à base de silice, ayant subi un lavage acide, comprend a) le lavage acide de la matrice oxyde mésoporeuse à base de silice, b) le traitement thermique de ladite matrice lavée issue de l'étape a) de façon à obtenir le support du catalyseur selon l'invention, c) le dépôt d'au moins un précurseur métallique d'au moins l'élément tantale à la surface dudit support obtenu à l'issue de l'étape b) et d) le traitement thermique du solide issu de l'étape c) de façon à obtenir le catalyseur .

Conformément à l'étape a) ou a') du procédé de préparation du catalyseur, la matrice oxyde mésoporeuse à base de silice est telle que décrite dans le présent texte de l'invention. En particulier, ladite matrice peut être commerciale ou bien synthétisée à façon selon les méthodes connues de l'Homme du métier. De plus, ladite matrice oxyde mésoporeuse à base de silice peut être utilisée directement sous forme de poudre ou bien déjà mise en forme, en particulier sous forme de poudre pastillée, concassée et tamisée, de billes, de pastilles, de granulés, ou d'extrudés (cylindres creux ou non, cylindres multilobés à 2, 3, 4 ou 5 lobes par exemple, cylindres torsadés), ou d'anneaux, etc., ces opérations de mise en forme étant réalisées par les techniques classiques connues de l'Homme du métier. De préférence, ladite matrice oxyde mésoporeuse à base de silice est obtenue sous forme d'extrudés de taille comprise entre 1 et 10 mm. Cependant, il n'est pas exclu que ladite matrice oxyde mésoporeuse à base de silice obtenue soit ensuite, par exemple, introduite dans un équipement permettant d'arrondir la surface, tel qu'un drageoir ou tout autre équipement permettant leur sphéronisation.

Conformément à l'étape a) du procédé de préparation du catalyseur, la matrice oxyde mésoporeuse à base de silice employée est lavée via la mise en contact de ladite matrice avec au moins un acide inorganique, dilué dans une solution aqueuse, et choisi parmi l'acide nitrique, l'acide sulfurique et l'acide chlorhydrique, l'acide nitrique et l'acide chlorhydrique étant préférés. La concentration en acide inorganique utilisé est comprise dans la gamme de 0,05 molaire (M) à 3 M et de façon préférée comprise dans la gamme de 0,1 M à 1 M. Ledit lavage acide peut être effectué dans des conditions statiques (par exemple en « batch ») ou continues (par exemple circulation dans une colonne de lavage avec ou sans recyclage partiel ou total de l'effluent). Selon l'invention, les conditions représentatives de ce lavage à l'acide sont une température comprise entre 0°C et 120°C, préférentiellement entre 15°et 80°C, et de manière très préférée entre 20°C et 70°C. Le temps de contact de la solution acide avec la matrice oxyde mésoporeuse varie entre 10 minutes et 10 heures et de manière préférée entre 30 minutes et 3 heures. Une opération optionnelle de rinçage à l'eau peut ensuite être mise en place de façon à éliminer l'excédent d'acidité. Celle-ci peut être réalisée dans les mêmes conditions que décrites ci-dessus. Cette opération est nécessaire dans les cas où l'acide ne peut pas être éliminé facilement lors des étapes b) et d) du procédé de préparation du catalyseur selon l'invention, ce dernier risquant alors d'être présent sur le catalyseur final. Ces conditions s'appliquent *mutatis mutandis* au solide issu de l'étape b') dans l'étape c') du procédé de préparation selon l'invention.

Cette étape de lavage acide est cruciale pour améliorer le potentiel d'un catalyseur de production de butadiène à partir d'un mélange comprenant au moins de l'éthanol, en ce sens qu'elle induit l'élimination de certaines impuretés potentielles du support et/ou modifie sa chimie de surface, ce qui conduit à une meilleure activité du catalyseur ainsi qu'à une amélioration de sa sélectivité.

A l'issue de cette opération et avant l'imprégnation de(s) l'élément(s) actif(s), le catalyseur peut contenir de faibles teneurs en sodium comprises entre 0 et 500 ppm, de préférence entre 0 et 300 ppm et de manière préférée entre 0 et 100 ppm.

Conformément à l'étape b) du procédé de préparation du catalyseur, la matrice oxyde mésoporeuse à base de silice lavée issue de l'étape a) du procédé de préparation du catalyseur subit au moins un traitement thermique afin de libérer la porosité de ladite matrice. Ce traitement correspond à un séchage de ladite matrice lavée. Ledit traitement est réalisé dans une gamme de température de 80°C à 300°C durant une période inférieure à 24 h. Ces conditions s'appliques *mutatis mutandis* à l'étape d').

Conformément à l'étape c) du procédé de préparation du catalyseur, au moins une étape de dépôt d'au moins un précurseur métallique d'au moins l'élément tantale à la surface dudit support obtenu à l'issue de l'étape b) est réalisé par toutes méthodes de synthèse connues de l'Homme du métier. Par exemple et de façon non exhaustive, les méthodes dites d'imprégnation à sec, d'imprégnation en excès, CVD (Chemical Vapor Deposition ou dépôt chimique en phase vapeur), CLD (Chemical Liquid Deposition ou dépôt chimique en phase liquide), etc. peuvent être employées. Pour exemple, dans le cas d'un dépôt réalisé par la méthode d'imprégnation à sec, l'étape c) du procédé de préparation du catalyseur est constituée des opérations unitaires suivantes : la dissolution d'au moins un précurseur d'au moins l'élément tantale dans un volume de solution correspondant au volume poreux du support obtenu à l'issue de l'étape b) du procédé de préparation selon l'invention, l'imprégnation de ladite solution à la surface dudit support et la maturation éventuelle du solide ainsi obtenu dans une atmosphère et à une température contrôlées de façon à favoriser la dispersion d'au moins dudit précurseur utilisé sur l'ensemble de la surface du support.

Conformément à l'étape c) du procédé de préparation du catalyseur, le précurseur métallique d'au moins l'élément tantale est tout composé comprenant au moins l'élément tantale et pouvant libérer cet élément en solution sous forme réactive. Ainsi, les précurseurs d'au moins l'élément tantale sont avantageusement des sels inorganiques et des précurseurs alcoxydes. Les sels inorganiques sont choisis dans le groupe constitué par les halogénures, les nitrates, les sulfates, les phosphates, les hydroxydes, les carbonates, les carboxylates, les alcoolates, et des combinaisons de deux ou plusieurs de ceux-ci, plus préférablement choisis dans le groupe constitué par les chlorures, les nitrates, les carboxylates, les alcoolates, et des combinaisons de deux ou plusieurs de ceux-ci. Les précurseurs alcoxydes ont par exemple pour formule M(OR)$_n$ où M = Ta. et R = éthyle, isopropyle, n-butyle, s-butyle, t-butyle, etc. ou un précurseur chélaté tel que X(C$_5$H$_8$O$_2$)$_n$, avec n = 3 ou 4. Par exemple, les précurseurs préférés du tantale sont le pentachlorure de tantale et le pentaéthanoate de tantale qui peuvent être utilisés avec la plupart des solvants organiques. Les conditions de l'étape c) s'appliquent *mutatis mutandis* à l'étape a').

Conformément à l'étape d) du procédé de préparation du catalyseur, le solide issu de l'étape c) du procédé de préparation du catalyseur subit alors au moins une étape de traitement thermique de façon à obtenir le catalyseur . Ce traitement correspond à un séchage suivi d'une calcination. La phase de séchage est réalisée en faisant circuler sur le solide un gaz à une température comprise entre 80 et 150°C, pendant une durée comprise entre 1 et 24 heures. Ladite étape de séchage est effectuée sous air. La phase de calcination est réalisée en faisant circuler sur le solide séché un gaz à une température comprise entre 450 et 600°C pendant une durée comprise entre 1 et 6 h et de préférence comprise entre 2 et 4 h. Ladite étape de calcination est mise en œuvre sous un flux gazeux comprenant de l'oxygène. Les conditions de l'étape d) s'appliquent *mutatis mutandis* à l'étape b').

Dans le cas où la matrice oxyde mésoporeuse à base de silice nécessaire à l'étape a) ou a') du procédé de préparation du catalyseur est utilisée sous la forme d'une poudre non mise en forme, le catalyseur, lui-même non mis en forme à l'issue de l'étape d) ou d') du procédé de préparation selon l'invention, peut être mis en forme sous forme de poudre pastillée, concassée, tamisée, de billes, de pastilles, de granulés, ou d'extrudés (cylindres creux ou non, cylindres multilobés à 2, 3, 4 ou 5 lobes par exemple, cylindres torsadés), ou d'anneaux, etc., ces opérations de mise en forme étant réalisées par les techniques classiques connues de l'Homme du métier. De préférence, ledit catalyseur est obtenu sous forme d'extrudés de taille comprise entre 1 et 10 mm. Cependant, il n'est pas exclu que lesdits matériaux obtenus soient ensuite, par exemple introduits dans un équipement permettant d'arrondir leur surface, tel qu'un drageoir ou tout autre équipement permettant leur sphéronisation.

Lors de ladite opération de mise en forme, le catalyseur peut éventuellement être mélangé avec au moins un matériau oxyde poreux ayant le rôle de liant de façon à générer les propriétés physiques du catalyseur adéquates au procédé (résistance mécanique, résistance à l'attrition, etc.).

Ledit matériau oxyde poreux est préférentiellement un matériau oxyde poreux choisi dans le groupe formé par la silice, la magnésie, les argiles, l'oxyde de titane, l'oxyde de lanthane, l'oxyde de cérium, les phosphates de bore et un mélange d'au moins deux des oxydes cités ci-dessus. On peut utiliser également les titanates, par exemple les titanates de zinc, nickel, cobalt. Il est encore possible d'employer des argiles simples, synthétiques ou naturelles de type phyllosilicate 2:1 dioctaédrique ou phyllosilicate 3:1 trioctaédrique telles que la kaolinite, l'antigorite, la chrysotile, la montmorillonnite, la beidellite, la vermiculite, le talc, l'hectorite, la saponite, la laponite. Ces argiles peuvent être éventuellement délaminées. Les divers mélanges utilisant au moins deux des composés cités ci-dessus conviennent également pour assurer le rôle de liant.

De façon très préférée, le liant utilisé est de nature silicique. Pour exemple et de façon non exhaustive, le dit liant silicique peut être sous le forme de poudres ou de solutions colloïdales.

De préférence, ledit catalyseur comprend de 5 à 60% poids, et de manière préférée entre 10 et 30% poids de liant silicique, les pourcentages poids étant exprimés par rapport à la masse totale dudit catalyseur.

Éventuellement, au moins un adjuvant organique est également mélangé au cours de ladite étape de mise en forme. La présence dudit adjuvant organique facilite la mise en forme par extrusion. Ledit adjuvant organique peut avantageusement être choisi parmi le méthylcellulose, l'hydroxypropylméthylcellulose, l'hydroxyéthylcellulose, le carboxyméthylcellulose et l'alcool polyvinylique. La proportion dudit adjuvant organique est avantageusement comprise entre 0 et 20% en poids, de préférence entre 0 et 10% en poids et de manière préférée entre 0 et 7% en poids, par rapport à la masse totale dudit matériau mis en forme.

Dans ce cas particulier d'une mise en forme du catalyseur consécutive à l'étape d) de traitement thermique du procédé de préparation, ladite étape de post-traitement est reconduite après mise en forme.

Pour finir, l'ajout éventuel d'au moins un élément des groupes 2, 3, 4, 5, 11 et 12 du tableau périodique peut être réalisé selon toutes les méthodes connues de l'Homme du métier et à n'importe quelle quelles étapes de synthèse et/ou de mise en forme de la matrice oxyde mésoporeuse à base de silice utilisée ou du catalyseur.

Les deux procédés selon l'invention ne diffèrent que par l'ordre dans lequel est réalisé l'étape de lavage, à savoir soit dans l'étape a) préalablement au dépôt du précurseur métallique d'au moins l'élément tantale, soit dans l'étape c') postérieurement au dépôt dudit précurseur métallique. Ces deux procédés sont des alternatives permettant de résoudre le même problème technique de préparation d'un catalyseur présentant des performances améliorées.

Les paramètres texturaux précités sont déterminés par la technique d'analyse dite de « Volumétrie à l'azote » qui correspond à l'adsorption physique de molécules d'azote dans la porosité du matériau via une augmentation progressive de la pression à température constante. On entend par surface spécifique, la surface spécifique B.E.T. (S$_{BET}$ en m$^2$/g) déterminée par adsorption d'azote conformément à la norme ASTM D 3663-78 établie à partir de la méthode BRUNAUER-EMMETT-TELLER décrite dans le périodique « The Journal of American Society », 1938, 60, 309. La distribution poreuse représentative d'une population de mésopores est déterminée par le modèle Barrett-Joyner-Halenda (BJH). L'isotherme d'adsorption - désorption d'azote selon le modèle BJH ainsi obtenue est décrite dans le périodique « The Journal of American Society », 1951, 73, 373, écrit par E. P. Barrett, L. G. Joyner et P. P. Halenda. Le volume poreux V est défini comme la valeur correspondant au volume observé pour la pression partielle $P/P^0_{max}$ de l'isotherme d'adsorption - désorption d'azote. Dans l'exposéqui suit , on entend par volume adsorption azote, le volume mesuré pour $P/P^0_{max} = 0,99$, pression pour laquelle il est admis que l'azote a rempli tous les pores. Dans l'exposé qui suit, le diamètre des mésopores $\Phi$ de l'oxyde mixte selon l'invention est déterminé par la formule $4000.V/S_{BET}$.

Dans l'exposé qui suit, la distribution poreuse mesurée par porosimétrie au mercure est déterminée par intrusion au porosimètre à mercure selon la norme ASTM D4284-83 à une pression maximale de 4000 bar (400 MPa), utilisant une tension de surface de 484 dyne/cm et un angle de contact de 140°. L'angle de mouillage a été pris égal à 140° en suivant les recommandations de l'ouvrage "Techniques de l'ingénieur, traité analyse et caractérisation, P 1050-5, écrits par Jean Charpin et Bernard Rasneur ».

On fixe à 0,2 MPa la valeur à partir de laquelle le mercure remplit tous les vides intergranulaires, et on considère qu'au-delà le mercure pénètre dans les pores du solide.

Afin d'obtenir une meilleure précision, la valeur du volume poreux total correspond à la valeur du volume poreux total mesuré par intrusion au porosimètre à mercure mesurée sur l'échantillon moins la valeur du volume poreux total mesuré par intrusion au porosimètre à mercure mesurée sur le même échantillon pour une pression correspondant à 30 psi (environ 0,2 MPa).

Le volume macroporeux du catalyseur est défini comme étant le volume cumulé de mercure introduit à une pression comprise entre 0,2 MPa et 30 MPa, correspondant au volume contenu dans les pores de diamètre apparent supérieur à 50 nm.

Le volume mésoporeux du catalyseur est défini comme étant le volume cumulé de mercure introduit à une pression comprise entre 30 MPa et 400 MPa, correspondant au volume contenu dans les pores de diamètre apparent compris entre 2 et 50 nm.

Le volume des micropores est mesuré par porosimétrie à l'azote. L'analyse quantitative de la microporosité est effectuée à partir de la méthode "t" (méthode de Lippens-De Boer, 1965) qui correspond à une transformée de l'isotherme d'adsorption de départ comme décrit dans l'ouvrage « Adsorption by powders and porous solids. Principles, methodology and applications » écrit par F. Rouquérol, J. Rouquérol et K. Sing, Academic Press, 1999.

On définit également le diamètre médian des mésopores (Dp en nm) comme étant un diamètre tel que tous les pores de taille inférieure à ce diamètre constituent 50% du volume mésoporeux, mesuré par porosimétrie au mercure.

L'utilisation d'une matrice oxyde mésoporeuse à base de silice lavée comme support d'un catalyseur comprenant au moins l'élément tantale pour la conversion d'une charge comprenant au moins de l'éthanol en butadiène, se traduit par des avantages de performances significatifs en termes d'activité catalytique, de niveau de conversion obtenus à une température de réaction donnée et/ou de sélectivité. Les conditions représentatives pour cette réaction sont une température comprise entre 300 et 400°C, de préférence entre 320°C et 380°C, une pression comprise entre 0,15 et 0,5 MPa, de préférence entre 0,15 et 0,3 MPa, une vitesse spatiale comprise entre 0,5 et 5 $h^{-1}$ de préférence entre 1 et 4 $h^{-1}$. Lorsque la charge traitée comprend également de l'acétaldéhyde, le ratio massique éthanol/acétaldéhyde est compris entre 1 et 30, de préférence entre 2 et 10. La vitesse spatiale est définie comme le rapport entre le débit massique de charge et la masse de catalyseur.

L'invention est illustrée au moyen des exemples qui suivent.

## Exemples

### Définition des termes

$$\text{pph (g/g}_{Ta}\text{/h)} : pph = \frac{\text{débit massique de charge } (\frac{g}{h})}{\text{masse de tantale } (gTa)}$$

Productivité ($g_c/g_{Ta}$/h) :

$$productivité = \frac{\text{débit massique de carbone appartenant au butadiène } (\frac{gc}{h})}{\text{masse de tantale présent sur le catalyseur } (gTa)}$$

Sélectivité (%C) :

$$sélectivité = \frac{débit\ massique\ de\ carbone\ appartenant\ au\ butadiène\ (\frac{gc}{h})}{débit\ massique\ de\ carbone\ appartenant\ à\ la\ charge\ convertie}$$

**Exemple 1 - Préparation de la silice**

A une solution contenant 55 ml de tétraéthylorthosilicate (TEOS, $Si(OCH_2CH_3)_4$) et 150 ml d'éthanol sont ajoutés 12,5 ml d'une solution d'acide nitrique à 68% (volumique) à température ambiante. L'ensemble est laissé sous agitation pendant 30 min. 50 ml d'une solution d'ammoniaque à 14% (volumique) sont alors ajoutés. Le système se trouble et un gel se forme. 19 ml d'éthanol sont alors ajoutés pour permettre une agitation supplémentaire durant 3 heures. Le gel final est filtré, lavé à l'éthanol puis séché à 100°C durant 24 heures. La poudre de silice obtenue est alors calcinée sous air à 550°C pendant 4 heures.

Les caractéristiques des matrices oxydes mésoporeuses utilisées dans les exemples sont récapitulées ci-dessous.

Tableau 1

| Matrice oxyde mésoporeuse | Surface BET ($m^2$/g) | Diamètre moyen des pores (nm) | VRE (ml/g) | Présence des métaux alcalins |
|---|---|---|---|---|
| Merck 7734 | 550 | 6 | 0,8 | >500 ppm |
| Davisil 636 | 507 | 7 | 1 | >500 ppm |
| Davisil 646 | 300 | 15 | 1,2 | >500 ppm |
| Silice synthétisée Selon l'exemple 1 | 690 | 15 | 1,5 | < 100 ppm |

**Exemple 2 - Lavage de la matrice oxyde mésoporeuse**

Les différentes matrices oxydes mésoporeuses décrites dans le tableau 1 (granulométrie : 250-500 $\mu m$) sont placés sur un fritté n°4 sur lequel est passé pendant 1 heure une solution de lavage. Le volume de solution utilisé représente environ 5 fois le volume occupé par la matrice oxyde mésoporeuse. Les solides lavés sont rincés avec un volume équivalent d'eau distillée pendant 1 heure supplémentaire puis sont placés en étuve à 115°C pendant au moins 4 heures.

Les caractéristiques des matrices oxydes mésoporeuses après lavages sont récapitulées dans le tableau 2.

Tableau 2

| Support silicique | Surface BET ($m^2$/g) | Diamètre moyen des pores (nm) | Volume poreux (ml/g) | Présence des métaux alcalins |
|---|---|---|---|---|
| Merck 7734 | 570 | 6 | 0,8 | <500 ppm |
| Davisil 636 | 512 | 7 | 1 | <500 ppm |
| Davisil 646 | 320 | 14 | 1,2 | <500 ppm |
| Silice synthétisée dans l'exemple 1 | 620 | 13 | 1,5 | < 100 ppm |

Le lavage acide décrit ci-dessus a permis de diminuer les teneurs en alcalins en dessous de 1000 ppm pour l'ensemble des supports siliciques et a légèrement modifié la texture des supports.

**Exemple 3 - Imprégnation à sec des supports pour le dépôt du tantale**

Le pentaéthoxyde de tantale ($Ta(OCH_2CH_3)_5$) (dont la quantité est calculée à partir de la teneur en Ta à déposer sur le support) est dilué dans une solution d'éthanol (dont la quantité est proportionnelle au volume poreux du support silicique). Cette solution est rapidement ajoutée au goutte à goutte et mélangée au support silice jusqu'à observer une mouillabilité de la surface de ce dernier (imprégnation à sec). Le solide est alors placé dans une atmosphère saturée en éthanol durant 3 heures, séché à 100°C durant 24 heures. Le catalyseur est obtenu par calcination du solide séché sous air à 550°C pendant 4 heures.

**Exemple 4 - Imprégnation à sec pour le dépôt de niobium**

L'oxalate d'ammonium et de niobium pentahydraté (dont la quantité est calculée à partir de la teneur en Nb à déposer sur le support) est dilué dans une solution aqueuse (dont la quantité est proportionnelle au volume poreux du support silicique). Cette solution est rapidement ajoutée au goutte à goutte et mélangée à la silice jusqu'à observer une mouillabilité de la surface de ce dernier (imprégnation à sec). Le solide est alors placé dans une atmosphère saturée en eau durant 3 heures, séché à 100°C durant 24 heures. Le catalyseur est obtenu par calcination du solide séché sous air à 550°C pendant 4 heures.

**Exemple 5 - Imprégnation à sec pour le dépôt de zirconium**

Le chlorure de zirconyle (dont la quantité est calculée à partir de la teneur en Zr à déposer sur le support) est dilué dans une solution aqueuse (dont la quantité est proportionnelle au volume poreux du support silicique). Cette solution est rapidement ajoutée au goutte à goutte et mélangée à la silice jusqu'à observer une mouillabilité de la surface de ce dernier (imprégnation à sec). Le solide est alors placé dans une atmosphère saturée en eau durant 3 heures, séché à 100°C durant 24 heures. Le catalyseur est obtenu par calcination du solide séché sous air à 550°C pendant 4 heures.

Le tableau 3 présente un récapitulatif des catalyseurs préparés.

| catalyseur | Selon l'invention O/N | Silice de départ | Solution de lavage | Métal et teneur imprégnée |
|---|---|---|---|---|
| A | N | Davisil 636 | --- | 0,5% Ta |
| B | O | Davisil 636 | HCl 1N | 0.5% Ta |
| C | N | Davisil 636 | --- | 2% Ta |
| D | O | Davisil 636 | HCl 0,1N | 2% Ta |
| E | O | Davisil 636 | HCl 1N | 2% Ta |
| F | N | Davisil 646 | --- | 2% Ta |
| G | O | Davisil 646 | HCl 0,1N | 2% Ta |
| H | N | Merck 7734 | --- | 0,5% Ta |
| I | N | Merck 7734 | eau | 0,5% Ta |
| J | O | Merck 7734 | HCl 0,1N | 0,5% Ta |
| K | N | Silice synthétisée | --- | 2% Ta |
| L | O | Silice synthétisée | HCl 0,1N | 2% Ta |
| M | N | Davisil 636 | --- | 2% Nb |
| N | N | Davisil 636 | HCl 1N | 2% Nb |
| O | N | Davisil 636 | --- | 2% Zr |
| P | N | Davisil 636 | HCl 1N | 2% Zr |
| Q | O | 2%Ta/Davisil 636 | HCl 0,1N | --- |

Tableau 3

**Description de l'unité de test catalytique**

Le réacteur utilisé dans les exemples qui suivent consiste en un tube en acier inoxydable de 20 cm de long et de 10 mm de diamètre. Le réacteur est d'abord chargé avec du carborundum puis avec le catalyseur dilué dans du carborundum et enfin avec du carborundum. Le carborundum est inerte vis-à-vis de la charge et n'influe pas sur les résultats catalytiques ; il permet de positionner le catalyseur dans la zone isotherme du réacteur et de limiter les risques de problèmes de transfert de chaleur et de matière. La température du réacteur est contrôlée avec un four tubulaire à trois zones de chauffe. La charge liquide (mélange d'éthanol et d'acétaldéhyde dans une proportion R) est injectée via une pompe HPLC à double piston. Le flux liquide est vaporisé dans les lignes chauffées par un traceur avant d'entrer dans le réacteur et est homogénéisé par le passage dans un mélangeur statique. Les produits formés lors de la réaction sont maintenus en phase vapeur pour être analysés en ligne par chromatographie gazeuse (colonnes capillaires PONA et Carboxen 1010) pour permettre l'identification la plus précise des centaines de produits formés. Le catalyseur est activé in situ sous azote à la température de test. Les conditions opératoires spécifiques sont décrites dans les exemples suivants.

**Exemple 6 - Impact du lavage acide sur des catalyseurs comprenant du tantale**

Dans ce test, le ratio Ethanol/Acétaldéhyde de la charge est fixé à 2,6 (mol/mol), la température à 350°C et la pression à 0,15 MPa. Pour chaque catalyseur, le débit de charge est ajusté pour obtenir une pph constante de $250g/g_{Ta}/h$.

Les valeurs de sélectivité et de productivité carbone sont mesurés au niveau de ce point de fonctionnement.

L'impact du lavage acide sur des catalyseurs à base de tantale est démontré par les résultats récapitulés dans le tableau 4.

Tableau 4

| catalyseur | Selon l'invention O/N | Productivité butadiène (gC/gTa/h) | Gain en productivité (%) | sélectivité | Gain en sélectivité |
|---|---|---|---|---|---|
| A | N | 27 | --- | 66% | --- |
| B | O | 39 | +44% | 72% | +6 |
| C | N | 37 | --- | 72% | --- |
| D | O | 42 | +14% | 74% | +2 |
| E | O | 43 | +16% | 74% | +2 |
| F | N | 18 | --- | 65% | --- |
| G | O | 33 | +83% | 69% | +4 |
| H | N | 11 | --- | 57% | --- |
| I | N | 11 | 0% | 60% | +3 |
| J | O | 40 | +263% | 72% | +15 |
| K | N | 35 | --- | 73% | --- |
| L | O | 42 | +20% | 74% | +1 |

Le lavage acide de la matrice oxyde mésoporeuse avant imprégnation de l'élément tantale permet une amélioration de la productivité en butadiène et de la sélectivité du catalyseur pour toutes les matrices oxydes mésoporeuses utilisées par rapport aux catalyseurs ayant des teneurs identiques en tantale mais dont la matrice oxyde mésoporeuse n'a pas subi de lavage acide, ou pour lesquels la matrice oxyde mésoporeuse n'a subi qu'un lavage à l'eau.

**Exemple 7 - Impact du lavage acide sur des catalyseurs à base de niobium ou zirconium ne contenant pas l'élément tantale**

Tableau 5

| catalyseur | Selon l'invention O/N | Productivité butadiène (gC/gM/h) | Gain en productivité (%) | sélectivité | Gain en sélectivité |
|---|---|---|---|---|---|
| M | N | 33 | --- | 57% | --- |
| N | N | 40 | +21% | 45% | -12 |
| O | N | 60 | --- | 63% | --- |
| P | N | 72 | +20% | 63% | 0 |

Les catalyseurs M et N sont obtenus en imprégnant 2%Nb sur une silice Davisil 636. On constate que le lavage acide de la silice permet une amélioration de la productivité, mais que la sélectivité de ces catalyseurs ne contenant pas l'élément tantale est fortement dégradée.

Les catalyseurs O et P sont obtenus en imprégnant 2%Zr sur une silice Davisil 636. On constate une amélioration de la productivité, mais une absence d'effet sur la sélectivité du lavage acide de la silice sur ces catalyseurs ne contenant pas l'élément tantale.

C'est donc bien l'association du lavage acide et de la présence de l'élément tantale qui permet un gain de sélectivité, et également de productivité pour la production de 1,3-butadiène.

**Exemple 8** - Impact de l'ordre des étapes préparatoires sur des catalyseurs à base de tantale Impact de l'ordre des étapes préparatoires sur des catalyseurs à base de tantale

| catalyseur | Selon l'invention O/N | Productivité butadiène (gC/gM/h) | Gain en productivité (%) | sélectivité | Gain en sélectivité |
|---|---|---|---|---|---|
| C | N | 37 | --- | 72% | --- |
| D | O | 42 | +14% | 74% | +2 |
| Q | O | 39 | +7% | 74% | +2 |

Le lavage acide du solide après dépôt de la phase active à base de tantale permet également d'obtenir de meilleures performances. Ce résultat est surprenant car on aurait pu s'attendre à une altération de la phase active.

**Revendications**

1. Procédé de préparation d'un catalyseur comprenant au moins l'élément tantale, et au moins une matrice oxyde mésoporeuse à base de silice, la masse de tantale représentant entre 0,1 et 30% de la masse de ladite matrice oxyde mésoporeuse, comprenant au moins les étapes suivantes :

   a) au moins une étape de lavage acide d'une matrice oxyde mésoporeuse à base de silice comprenant avant lavage au moins 90% poids de silice, ladite matrice oxyde mésoporeuse à base de silice avant lavage étant une silice amorphe mésoporeuse à porosité non organisée sans micropores ayant une surface spécifique BET d'au moins 250 m$^2$/g, les pourcentages poids étant exprimés par rapport à la masse totale de la matrice oxyde mésoporeuse, ledit lavage étant réalisé via la mise en contact avec au moins un acide inorganique, choisi parmi l'acide nitrique, l'acide sulfurique et l'acide chlorhydrique, dilué dans une solution aqueuse à une concentration comprise dans la gamme de 0,05 M à 3 M, à une température comprise entre 0°C et 120°C et un temps de contact de ladite solution acide avec ladite matrice oxyde mésoporeuse compris entre 10 min et 10 h,
   b) au moins une étape de traitement thermique de ladite matrice lavée issue de l'étape a), ledit traitement thermique étant un séchage réalisé à une température de 80 à 300°C durant une période inférieure à 24 h, de façon à obtenir un support de catalyseur,
   c) au moins une étape de dépôt d'au moins un précurseur métallique d'au moins l'élément tantale sur la surface dudit support obtenu à l'issue de l'étape b) et
   d) au moins une étape de traitement thermique du solide issu de l'étape c), ledit traitement thermique étant un séchage suivi d'une calcination, le séchage étant réalisé sous circulation d'air à une température comprise entre 80 et 150°C pendant une durée comprise entre 1 et 24 heures, et la calcination étant réalisée sous circulation d'un flux gazeux comprenant de l'oxygène, à une température comprise entre 450 et 600°C, pendant une durée comprise entre 1 et 6 heures.

2. Procédé de préparation d'un catalyseur comprenant au moins l'élément tantale, et au moins une matrice oxyde mésoporeuse à base de silice, la masse de tantale représentant entre 0,1 et 30% de la masse de ladite matrice oxyde mésoporeuse, comprenant au moins les étapes suivantes :

   a') au moins une étape de dépôt d'au moins un précurseur métallique d'au moins l'élément tantale sur la surface d'une matrice oxyde mésoporeuse à base de silice comprenant avant lavage au moins 90% poids de silice, ladite matrice oxyde mésoporeuse à base de silice avant lavage étant une silice amorphe mésoporeuse à porosité non organisée sans micropores ayant une surface spécifique BET d'au moins 250 m$^2$/g, les pourcentages poids étant exprimés par rapport à la masse totale de la matrice oxyde mésoporeuse,

b') au moins une étape de traitement thermique du solide issu de l'étape a'), ledit traitement thermique étant un séchage suivi d'une calcination, le séchage étant réalisé sous circulation d'air à une température comprise entre 80 et 150°C pendant une durée comprise entre 1 et 24 heures, et la calcination étant réalisée sous circulation d'un flux gazeux comprenant de l'oxygène, à une température comprise entre 450 et 600°C, pendant une durée comprise entre 1 et 6 heures

c') au moins une étape de lavage acide du solide issu de l'étape b'), avec au moins un acide inorganique, choisi parmi l'acide nitrique, l'acide sulfurique et l'acide chlorhydrique, dilué dans une solution aqueuse à une concentration comprise dans la gamme de 0,05 M à 3 M, à une température comprise entre 0°C et 120°C et un temps de contact de ladite solution acide avec ledit solide compris entre 10 min et 10 h,

d') au moins une étape de traitement thermique du solide issu de l'étape c'), ledit traitement thermique étant un séchage réalisé à une température de 80 à 300°C durant une période inférieure à 24 h

3. Procédé selon l'une des revendications précédentes dans lequel ladite matrice oxyde mésoporeuse a, avant lavage, une surface spécifique comprise entre 250 m$^2$/g et 700 m$^2$/g.

4. Procédé selon l'une des revendications précédentes dans lequel ladite matrice oxyde mésoporeuse a, avant lavage, un diamètre moyen des pores d'au moins 4 nm, de préférence compris entre 4,5 et 50 nm et de manière préférée entre 4,5 et 20 nm.

5. Procédé selon l'une des revendications précédentes dans lequel la masse de tantale représente entre 0,3 et 10%, de préférence entre 0,5 et 5% et de manière très préférée entre 0,5 et 2%, de la masse de ladite matrice oxyde mésoporeuse.

6. Procédé selon la revendication 1 dans lequel la teneur en sodium du catalyseur à l'issue de l'étape a) est comprise entre 0 et 500 ppm

7. Procédé selon la revendication 1 ou 2 dans lequel la matrice oxyde mésoporeuse à base de silice avant lavage contient également, en plus de la silice, au moins un oxyde choisi dans le groupe constitué par la zircone, l'oxyde de titane, l'oxyde de bore, l'oxyde de lanthane, l'oxyde de cérium et leurs mélanges.

**Patentansprüche**

1. Verfahren zur Herstellung eines Katalysators, der mindestens das Element Tantal und mindestens eine mesoporöse Oxidmatrix auf Basis von Siliciumdioxid umfasst, wobei die Masse von Tantal zwischen 0,1 und 30 % der Masse der mesoporösen Oxidmatrix ausmacht, wobei das Verfahren mindestens die folgenden Schritte umfasst:

a) mindestens einen Schritt der Säurewäsche einer mesoporösen Oxidmatrix auf Basis von Siliciumdioxid, die vor der Wäsche mindestens 90 Gew.-% Siliciumdioxid umfasst, wobei es sich bei der mesoporösen Oxidmatrix auf Basis von Siliciumdioxid vor der Wäsche um ein mesoporöses amorphes Siliciumdioxid mit nicht organisierter Porosität ohne Mikroporen und einer spezifischen BET-Oberfläche von mindestens 250 m$^2$/g handelt, wobei die Gewichtsprozentanteile bezogen auf die Gesamtmasse der mesoporösen Oxidmatrix ausgedrückt sind, wobei die Wäsche durch Inkontaktbringen mit mindestens einer in einer wässrigen Lösung auf eine Konzentration im Bereich von 0,05 M bis 3 M verdünnten, aus Salpetersäure, Schwefelsäure und Salzsäure ausgewählten anorganischen Säure bei einer Temperatur zwischen 0 °C und 120 °C und einer Kontaktzeit der Säurelösung mit der mesoporösen Oxidmatrix zwischen 10 min und 10 h durchgeführt wird,

b) mindestens einen Schritt der Wärmebehandlung der gewaschenen Matrix aus Schritt a), wobei es sich bei der Wärmebehandlung um eine bei einer Temperatur von 80 bis 300 °C über einen Zeitraum von weniger als 24 h durchgeführte Trocknung handelt, wodurch ein Katalysatorträger erhalten wird,

c) mindestens einen Schritt der Abscheidung mindestens eines Metallvorläufers mindestens des Elements Tantal auf der Oberfläche des am Ende von Schritt b) erhaltenen Trägers und

d) mindestens einen Schritt der Wärmebehandlung des Feststoffs aus Schritt c), wobei es sich bei der Wärmebehandlung um eine Trocknung mit anschließender Calcinierung handelt, wobei die Trocknung unter Zirkulieren von Luft mit einer Temperatur zwischen 80 und 150 °C über einen Zeitraum zwischen 1 und 24 Stunden durchgeführt wird und die Calcinierung unter Zirkulieren eines Gasstroms, der Sauerstoff umfasst, bei einer Temperatur zwischen 450 und 600 °C über einen Zeitraum zwischen 1 und 6 Stunden durchgeführt wird.

2. Verfahren zur Herstellung eines Katalysators, der mindestens das Element Tantal und mindestens eine mesoporöse Oxidmatrix auf Basis von Siliciumdioxid umfasst, wobei die Masse von Tantal zwischen 0,1 und 30 % der Masse der mesoporösen Oxidmatrix ausmacht, wobei das Verfahren mindestens die folgenden Schritte umfasst:

a') mindestens einen Schritt der Abscheidung mindestens eines Metallvorläufers mindestens des Elements Tantal auf der Oberfläche einer mesoporösen Oxidmatrix auf Basis von Siliciumdioxid, der vor der Wäsche mindestens 90 Gew.-% Siliciumdioxid umfasst, wobei es sich bei der mesoporösen Oxidmatrix auf Basis von Siliciumdioxid vor der Wäsche um ein mesoporöses amorphes Siliciumdioxid mit nicht organisierter Porosität ohne Mikroporen und einer spezifischen BET-Oberfläche von mindestens 250 m$^2$/g handelt, wobei die Gewichtsprozentanteile bezogen auf die Gesamtmasse der mesoporösen Oxidmatrix ausgedrückt sind, b') mindestens einen Schritt der Wärmebehandlung des Feststoffs aus Schritt a'), wobei es sich bei der Wärmebehandlung um eine Trocknung mit anschließender Calcinierung handelt, wobei die Trocknung unter Zirkulieren von Luft mit einer Temperatur zwischen 80 und 150 °C über einen Zeitraum zwischen 1 und 24 Stunden durchgeführt wird und die Calcinierung unter Zirkulieren eines Gasstroms, der Sauerstoff umfasst, bei einer Temperatur zwischen 450 und 600 °C über einen Zeitraum zwischen 1 und 6 Stunden durchgeführt wird,

c') mindestens einen Schritt der Säurewäsche des Feststoffs aus Schritt b') mit mindestens einer in einer wässrigen Lösung auf eine Konzentration im Bereich von 0,05 M bis 3 M verdünnten, aus Salpetersäure, Schwefelsäure und Salzsäure ausgewählten anorganischen Säure bei einer Temperatur zwischen 0 °C und 120 °C und einer Kontaktzeit der Säurelösung mit der mesoporösen Oxidmatrix zwischen 10 min und 10 h,

d') mindestens einen Schritt der Wärmebehandlung des Feststoffs aus Schritt c'), wobei es sich bei der Wärmebehandlung um eine bei einer Temperatur von 80 bis 300 °C über einen Zeitraum von weniger als 24 h durchgeführte Trocknung handelt.

3. Verfahren nach einem der vorhergehenden Ansprüche, wobei die mesoporöse Oxidmatrix vor der Wäsche eine spezifische Oberfläche zwischen 250 m$^2$/g und 700 m$^2$/g aufweist.

4. Verfahren nach einem der vorhergehenden Ansprüche, wobei die mesoporöse Oxidmatrix vor der Wäsche einen mittleren Porendurchmesser von mindestens 4 nm, vorzugsweise zwischen 4,5 und 50 nm und bevorzugt zwischen 4,5 und 20 nm aufweist.

5. Verfahren nach einem der vorhergehenden Ansprüche, wobei die Masse von Tantal zwischen 0,3 und 10 %, vorzugsweise zwischen 0,5 und 5 % und besonders bevorzugt zwischen 0,5 und 2 % der Masse der mesoporösen Oxidmatrix ausmacht.

6. Verfahren nach Anspruch 1, wobei der Natriumgehalt des Katalysators am Ende von Schritt a) zwischen 0 und 500 ppm liegt.

7. Verfahren nach Anspruch 1 oder 2, wobei die mesoporöse Oxidmatrix auf Basis von Siliciumdioxid vor der Wäsche außerdem neben Siliciumdioxid mindestens ein Oxid aus der Gruppe bestehend aus Zirconiumdioxid, Titanoxid, Boroxid, Lanthanoxid, Ceroxid und Mischungen davon enthält.

**Claims**

1. Process for producing a catalyst comprising at least the element tantalum, and at least one silica-based mesoporous oxide matrix, the weight of tantalum representing between 0.1 and 30% of the weight of said mesoporous oxide matrix, comprising at least the following steps:

a) at least one step of acid washing of a silica-based mesoporous oxide matrix comprising, before washing, at least 90% by weight of silica, said silica-based mesoporous oxide matrix, before washing, being a mesoporous amorphous silica with a non-organized porosity with no micropores, having a BET specific surface area of at least 250 m$^2$/g, the weight percentages being expressed relative to the total weight of the mesoporous oxide matrix, said washing being carried out by means of bringing into contact with at least one inorganic acid, chosen from nitric acid, sulfuric acid and hydrochloric acid, diluted in an aqueous solution at a concentration within the range of from 0.05 M to 3 M, at a temperature of between 0°C and 120°C and a contact time of said acid solution with said mesoporous oxide matrix of between 10 min and 10 h,

b) at least one step of heat treatment of said washed matrix resulting from step a), said heat treatment being drying carried out at a temperature of from 80 to 300°C for a period of less than 24 h, so as to obtain a catalyst support,

c) at least one step of deposition of at least one metal precursor of at least the element tantalum on the surface of said support obtained at the end of step b), and

d) at least one step of heat treatment of the solid resulting from step c), said heat treatment being drying followed by calcination, the drying being carried out under circulation of air at a temperature between 80 and 150°C for a period of between 1 and 24 hours, and the calcination being carried out under circulation of a gas stream comprising oxygen, at a temperature between 450 and 600°C, for a period of between 1 and 6 hours.

2. Process for producing a catalyst comprising at least the element tantalum, and at least one silica-based mesoporous oxide matrix, the weight of tantalum representing between 0.1 and 30% of the weight of said mesoporous oxide matrix, comprising at least the following steps:

a') at least one step of deposition of at least one metal precursor of at least the element tantalum on the surface of a silica-based mesoporous oxide matrix comprising, before washing, at least 90% by weight of silica, said silica-based mesoporous oxide matrix, before washing, being a mesoporous amorphous silica with a non-organized porosity with no micropores, having a BET specific surface area of at least 250 $m^2/g$, the weight percentages being expressed relative to the total weight of the mesoporous oxide matrix,

b') at least one step of heat treatment of the solid resulting from step a'), said heat treatment being drying following by calcination, the drying being carried out under circulation of air at a temperature between 80 and 150°C for a period of between 1 and 24 hours, and the calcination being carried out under circulation of a gas stream comprising oxygen, at a temperature between 450 and 600°C, for a period of between 1 and 6 hours,

c') at least one step of acid drying of the solid resulting from step b'), with at least one inorganic acid, chosen from nitric acid, sulfuric acid and hydrochloric acid, diluted in an aqueous solution at a concentration in the range of from 0.05 M to 3 M, at a temperature of between 0°C and 120°C and a contact time of said acid solution with said solid of between 10 min and 10 h,

d') at least one step of heat treatment of the solid resulting from step c'), said heat treatment being drying carried out at a temperature of from 80 to 300°C for a period of less than 24 h.

3. Process according to either of the preceding claims, in which said mesoporous oxide matrix has, before washing, a specific surface area between 250 $m^2/g$ and 700 $m^2/g$.

4. Process according to one of the preceding claims, in which said mesoporous oxide matrix has, before washing, a mean pore diameter of at least 4 nm, preferably of between 4.5 and 50 nm and preferably between 4.5 and 20 nm.

5. Process according to one of the preceding claims, in which the weight of tantalum represents between 0.3 and 10%, preferably between 0.5 and 5% and very preferably between 0.5 and 2%, of the weight of said mesoporous oxide matrix.

6. Process according to Claim 1, in which the sodium content of the catalyst at the end of step a) is between 0 and 500 ppm.

7. Process according to Claim 1 or 2, in which the silica-based mesoporous oxide matrix, before washing, also contains, in addition to the silica, at least one oxide chosen from the group consisting of zirconia, titanium oxide, boron oxide, lanthanum oxide and cerium oxide, and mixtures thereof.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2421361 A
- US 2374433 A
- US 2436125 A
- US 2438464 A
- US 2357855 A
- US 2447181 A
- WO 2014199349 A
- WO 2014180778 A
- WO 2014049158 A
- WO 2013125389 A
- WO 2012015340 A
- WO 2014061917 A
- US 2011196185 A

**Littérature non-brevet citée dans la description**

- **B. B. CORSON ; H. E. JONES ; C. E. WELLING ; J. A. HINCKLEY ; E. E. STAHLY.** *Ind. Eng. Chem.,* 1950, vol. 42 (2), 359-373
- *Ind. Eng. Chem.,* 1949, vol. 41, 1012-1017
- **W. J. TOUSSAINT ; J. T. DUNN ; D. R. JACKSON.** *Industrial and Engineering Chemistry,* 1947, vol. 39 (2), 120-125
- **2012 ; JONATHAN BURLA ; ROSS FEHNEL ; PHILIP LOUIE ; PETER TERPELUK.** TWO-STEP PRODUCTION OF 1,3-BUTADIENE FROM ETHANOL. université de Pennsylvanie
- **W. J. TOUSSAINT ; J. T. DUNN.** *Carbide and Carbon Chemical Corporation,* 1947
- *Ind. Eng. Chem.,* 1950, vol. 42 (2), 359-373
- **J. NAWROCKI.** Silica Surface Controversies, Strong Adsorption Sites, their Blockage and Removal. Part I. *Chromatographia,* 1991, vol. 31 (3/4
- **K. S. W. SING ; D. H. EVERETT ; R. A. HAUL ; L. MOSCOU ; J. PIEROTTI ; J. ROUQUEROL ; T. SIEMIENIEWSKA.** *Pure Appl. Chem.,* 1985, vol. 57, 603
- *The Journal of American Society,* 1938, vol. 60, 309
- **E. P. BARRETT ; L. G. JOYNER ; P. P. HALENDA.** *The Journal of American Society,* 1951, vol. 73, 373
- **JEAN CHARPIN ; BERNARD RASNEUR.** *Techniques de l'ingénieur, traité analyse et caractérisation,* 1050-5
- **F. ROUQUÉROL ; J. ROUQUÉROL ; K. SING.** Adsorption by powders and porous solids. Principles, methodology and applications. Academic Press, 1999